# EUROPEAN PATENT SPECIFICATION

(11) **EP 2 006 393 B1**
(45) Date of publication and mention of the grant of the patent: **18.05.2011**
(21) Application number: 07730387.3
(22) Date of filing: 13.03.2007
(51) Int. Cl.: C12Q 1/68

(54) **METHOD FOR ANALYSING NUCLEIC ACIDS**
VERFAHREN ZUR ANALYSE VON NUKLEINSÄUREN
PROCEDE D'ANALYSE D'ACIDES NUCLEIQUES

(30) Priority: 14.03.2006 ES 200600703
(43) Date of publication of application: 24.12.2008
(73) Proprietor: Oryzon Genomics, S.A., 08028 Barcelona (ES)
(72) Inventor: MAES, Tamara, 08028 Barcelona (ES); AIBAR DURAN, Elena, 08028 Barcelona (ES)
(74) Representative: Vossius & Partner
(86) International application number: PCT/ES2007/000146
(87) International publication number: WO 2007/104816

(56) References cited:
- WO-A-00/28081
- WO-A-00/61801
- WO-A-98/12352
- WO-A-03/016483
- WO-A1-00/17390
- WO-A2-2004/081183
- US-A- 5 955 276
- US-A- 6 045 994
- HUGHES S. ET AL.: 'The use of whole genome amplification in the study of human disease' PROG. BIOPHYS. MOL. BIOL. vol. 88, no. 1, 2005, pages 173 - 189, XP004651876
- LIU C.L. ET AL.: 'Development and validation of a T7 based liner amplification for genomic DNA' BMC GENOMICS vol. 4, 2003, page 19, XP021014452
- KLEIN C.A. ET AL.: 'Comparative genomic hybridization, loss of heterozygosity, and DNA sequence analysis of single cells' PROC. NATL. ACAD. SCI. USA vol. 96, 1999, pages 4494 - 4499, XP002144872
- GUILLAUD-BATAILLE M. ET AL.: 'Detecting single DNA copy number variations in complex genomes using one nanogram of starting DNA and BAC-array CGH' NUCLEIC ACIDS RES. vol. 32, no. 13, 2004, page E112, XP008129740

## Description

### FIELD OF THE INVENTION

The present invention relates to the field of molecular biology. In particular, the object of the present invention is a method of analyzing nucleic acids that can be used to determine the presence of variations in the genome of an organism, as regards both the sequence and the number of copies of a gene.

### BACKGROUND OF THE INVENTION

One of the techniques currently used to analyze changes in the number of copies of a gene in a genome is the method known as comparative genomic hybridization (CGH), which makes it possible to detect large chromosomal changes that take place in cells, including loss, duplication, and translocation of DNA from one cell to another.

With the development of DNA microarrays (also called DNA chips), these have been rapidly integrated into genome mapping studies, with the result that better resolution and sensitivity levels in the comparative analysis of genomic DNA and a greater reproductive capacity are being obtained, allowing reliable detection of alterations at individual gene level.

Thanks to its versatility. DNA microarray technology has applications in the fields of transcriptomics, genetics, and epigenetics. Accordingly, different protocols have been developed for labeling RNA and DNA samples in order to be able to perform bulk parallel analyses.

Theoretically, differences in signal intensity distribution should be observed when hybridizing to DNA microarrays according to whether the hybridized sample is RNA or DNA. in a cell, genes are expressed differentially, so that the various species of RNA found in a sample of total RNA can exhibit differences in expression levels of up to 4 orders of magnitude. In consequence, the hybridization signals of labeled RNA or aRNA samples cover a similar range of signal intensities (i.e. some 4 orders of magnitude) for probes on the microarray surface.

On the other hand, apart from repetitive DNA or duplicated or missing fragments of DNA in individual samples, the prevalence of the various DNA fragments in a genomic DNA sample is identical, and it would therefore be expected for the variation in signal intensity between the different probes on the microarray surface to be substantially smaller and to be restricted to small variations in the labeling efficiency of the various DNA fragments or to variations in the hybridization efficiency between the various labeled fragments and the probes on the microarray surface.

However, analysis of the signal distribution obtained from the various published protocols of whole-genomic and subgenomic hybridization reveals that the signal intensity distribution for the probes on the microarray surface is similar to that obtained in gene expression analysis, even when taking ultimate care in the probe selection procedure.

Labeling protocols that have been used for genomic studies include:
- genome fragmentation using DNase I and end-labeling with terminal transferase using labeled UTP (Borevitz et al., 2003. Large-scale identification of single-feature polymorphisms in complex genomes. Genome Research 13:513-523; Winzeler et al., 1998. Direct allelic variation scanning of the yeast genome. Science 281:1194-97).
- random labeling with primers (optionally after digestion with a restriction enzyme to generate smaller fragments) using labeled dNTPs. (Pollack et al., 1999. Genome-wide analysis of DNA copy-number changes using cDNA microarrays. Nature Genetics 23: 41-46).
- subgenomic amplification by digestion with one or more restriction enzymes, adapter binding, and amplification using adapter-based primers, followed by end-labeling with terminal transferase using labeled UTP (Maitra et al., 2005. Genomic alterations in cultured human embryonic stem cells. Nature Genetics 37(10): 1099-1103).
- subgenomic amplification by digestion with one or more restriction enzymes, adapter binding, and amplification using adapter-based primers labeled at one end.

All of these protocols generate signals distributed over 3-4 orders of magnitude, i.e. within the overall detection range of scanners currently in use. It is not yet known why this signal intensity distribution occurs, though this variation cannot be entirely explained by the labeling method, by differences in the thermodynamic characteristics of the probes on the surface, or by variations in the scanning process and must, therefore, be caused by deviations occurring in the labeling method.

WO0028081 discloses a process for the detection of polymorphisms whereby at least one restriction enzyme is used to generate restriction fragments from genomic. DNA. Subsequently adaptor segments are ligated to the ends of the restriction fragments and the fragments are amplified using primer-directed amplification wherein the amplification primers are labelled. The resulting fragments are hybridized to a bioarray for subsequent identification of potential variants, the latter revealing the potential polymorphisms / SNPs present in the sample.

WO03016483, discloses the introduction of labels during RNA-transcription however without disclosing the beneficiary effect and particular advantages found in the underlying invention in context of the analysis of genomic variations.

For this reason, attempts have been made to improve the labeling method, directed at reducing the amplitude of the signal intensity range (Lieu et al., 2005. Development of a DNA-labeling system for array based comparative genomic hybridization. J. Biom. Tech. 16:104-111).

The distribution of signal intensities over a broad range has several practical consequences:
- given that the signal-to-noise ratio deteriorates in the lowest signal range, a fraction of the signals is of insufficient quality for analysis. Signal intensity at the lower end of the spectrum can be improved by adding (up to a certain limit) more labeled DNA, but this causes the higher signals to move towards saturation and quantitative capacity is lost for these points.
- with some applications, including DNA mapping, it is desirable to be able to carry out bulk analysis of DNA samples, so that instead of analyzing a single sample in comparison with a control, the analysis is performed on a mixture of several samples, such that the level of hybridization in a mixture reflects, in comparison with a positive reference sample and a negative reference sample, the frequency with which a signal is present in the sample contained in the mixture. Typically, it would be desirable to be able to detect a signal that reflects a dilution one hundred times the signal of the positive reference sample. If, for example, the detectable signals are in the range between 60 and 60000, the positive reference signal should reach a value of at least 6000 and the negative reference sample should have a residual value appreciably below 60. With these applications, all the signal intensities should be comprised between 100 times the minimum clearly detectable signal and the highest detectable signals within the linear range of the scanner. Using current DNA labeling protocols, this criterion eliminates the majority of probes, because there are relatively few probes with an intensity greater than 100 times the background noise.
- with other applications, including analysis of the variations in the number of copies of a gene (such as CGH, for example), it is desirable to obtain in the middle of the spectrum the signal corresponding to the number of copies most frequently observed in order to allow duplications and deletions to be identified with maximum reliability.

However, with current protocols most of the points appear at low signal intensities, which makes observed changes in signal intensities difficult to interpret. This can be observed when analyzing data published in the literature such as, for example, the study published by Barrett *et al*. (Barrett MT et al. Comparative genomic hybridization using oligonucleotide microarrays and total genomic DNA. Proc Natl Acad Sci USA. 2004. Dec 21;101(51):17765-70).

This research group extracted genomic. DNA from human samples using Trizol (Invitrogen, USA) as the extraction reagent, in addition to phenol/chloroform purifications. 10 ng of this DNA was amplified by PCR using ϕ29 polymerase. Thereafter, this amplified DNA was digested with two restriction enzymes, Alul and Rsal, with an incubation time of 2 h at 37°C. The samples were labeled with 6 µg of DNA digested and purified with the Bioprime Labeling Kit (Invitrogen, USA), adding a nucleotide labeled with Cy3 or Cy5 fluorophore, following the steps recommended by said company.

Before hybridization, the labeled samples were denatured at 100°C for 1.5 min and incubated at 37°C for 30 min. The samples were hybridized in accordance with the recommendations of Agilent Technologies, incubating the reference sample and the test sample on the microarray overnight at 65°C. The microarrays were then washed in accordance with the Agilent protocol and scanned using an Agilent 2565AA DNA microarray scanner.

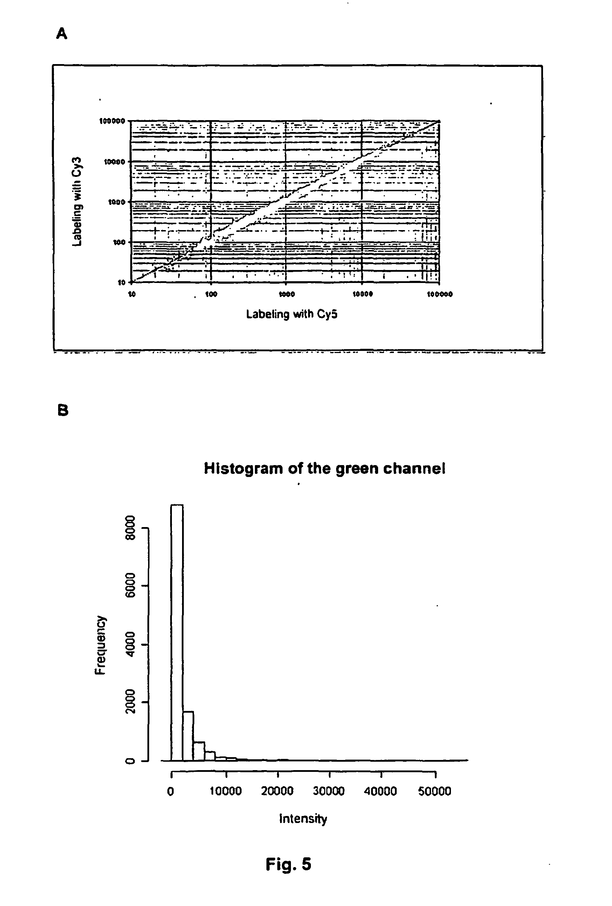

The graphic representation of the data corresponding to Dataset 14 in this publication are shown in Figures 5A and 5B, and the data analysis performed as described later can be found in Table 1. In order to be able to estimate the technical experimental scatter of the platform (i.e. the scatter of the signal levels of a repeated point, not the scatter of the entire data set obtained from the various probes), the oligonucleotides repeated several times in the microarray used in this document served as the controls. In particular, the probes used as controls are: ITGB3BP, EXO1, FLJ22116, IF2, CPS1, ST3GALVI, FLJ20432, HPS3, ARHH, SPP1, DKFZp762K2015, CENPE, CCNA2, ESM1, NLN, KIAA0372, LOX, RAD50, RAB6KIFL, FLJ20364, FLJ20624, SERPINE1, FLJ11785, FLJ11785, LOXL2, WRN, RAD54B, CML66, HAS2, MGC5254, MLANA, COL13A1, AD24, LM02, CD69, LOC51290, FLJ21908, MGC5585, KNTC1, TNFRSF11B, MGC5302, BAZ1A, AND-1, HIF1A, IFI27, FANCA, BRCA1, PMAIP1, HMCS, STCH, SERPIND1, and NSBP1. All these probes are repeated 10 times.

**Table 1**

| | Green channel | Red channel |
|---|---|---|
| Relative percentage scatter of probe signals | 174% | 173% |
| Relative percentage scatter of control signals. | 33% | 34% |
| Ratio between probe scatter and control scatter | 5.27 | 5.09 |

As can be seen from the data in Table 1, the probe signals exhibited a scatter more than 5 times the scatter exhibited by the controls, showing that there is a real scatter that is not due to the technical execution of the experiment. Moreover, it is observed graphically that the signals corresponding to the probes are distributed along the diagonal in the graph (Figure 5A, graph of the signal scatter obtained in the green channel and in the red channel) with a greater frequency at low signal intensities (Figure 5B, histogram reflecting the signal distribution). These results indicate that the particular combination of labeling protocol and hybridization to the collection of probes on the surface used in this publication introduces an undesirable variability that can affect the reliability of part of the results used.

In the present invention, a method is described for the analysis of genomic DNA, comprising DNA fractionation, adapter binding, and a step involving *in vitro* transcription of the samples using RNA polymerase. In this step, a set of RNA fragments is generated, with these RNA fragments being equivalent to the DNA fragments to be analyzed and being the ones to be hybridized to the DNA microarray oligonucleotides in order to carry out the analysis. The labeling of the samples may optionally be performed at this stage. The method according to the present invention makes it possible to significantly reduce the variability in the signal intensities of the analyzed samples.

### DESCRIPTION OF THE INVENTION

It is the object of the present invention to provide a method of analyzing nucleic acids comprising the following steps:
a) fragmentation of a sample of genomic DNA,
b) binding, at the ends of the DNA fragments obtained, of specific adapters compatible with the generated ends, wherein at least one of the bound adapters contains a functional promoter sequence,
c) amplification of the fragments obtained using specific adapter-based primers,
d) *in vitro* transcription of the amplified DNA fragments with an RNA polymerase capable of initiating the transcription from a promoter sequence contained in the adapters using a mixture of nucleotides (rNTPs),
e) hybridization to DNA microarray oligonucleotides and detection of hybridized fragments, and
f) quantitative comparison of the signals from the various samples analyzed.

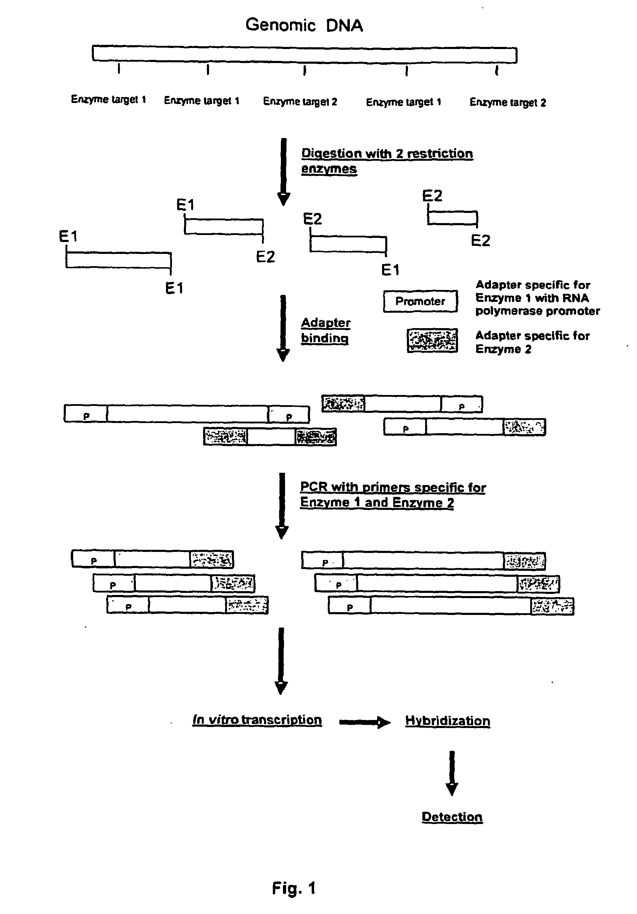

Figure 1 shows a diagram of an example of the steps constituting the method of the invention.

Fragmentation of a sample of genomic DNA can be carried out by chemical methods, such as, for example, treatment with hydrochloric acid, sodium hydroxide, hydrazine, etc.; by physical methods, including treatment with ionizing radiation, sonication, etc.; or by enzymatic methods, such as, for example, digestion with endonucleases, such as restriction enzymes. In one embodiment of the invention, fragmentation is accomplished by digestion with at least one restriction enzyme. In another embodiment of the invention, fragmentation is accomplished by digestion with two restriction enzymes.

The method of the present invention can be used for analyzing any sample of genomic DNA isolated from any organism wherein the study of the presence of variations in the genome is desired. Said method can be applied, among other things, to the bulk analysis of single-feature polymorphisms (SFP), comparative genomic hybridization (CGH), which makes it possible to determine the deletion of a gene or a fragment thereof, or the presence of two or more copies of a gene or fragments thereof, genetic mapping on the basis of analyses of individuals or by bulked segregant analysis, identification of single nucleotide polymorphism (SNP), localization of transposons, chromatin immunoprecipitation (ChiP-on-chip), etc.

The term microarray or DNA microarray refers to a collection of multiple oligonucleotides immobilized on a solid substrate, wherein each oligonucleotide is immobilized in a known position, such that each of the multiple oligonucleotides can be detected separately. The substrate may be solid or porous, planar or not planar, unitary or distributed. DNA microarrays on which the hybridization and detection are accomplished by the method of the present invention can be manufactured with oligonucleotides deposited by any process or with oligonucleotides synthesized *in situ* photolithography or by any other process.

The term probe refers to the oligonucleotides immobilized on the solid substrate with which hybridization of the nucleic acids to be analyzed takes place.

In one embodiment of the invention, detection of the hybridized fragments is accomplished on the basis of the direct quantification of the amount of hybridized sample on the DNA probes contained in the DNA microarray. Said direct quantification can be accomplished using techniques that include, but are not limited to, atomic force microscopy (AFM), scanning tunneling microscopy (STM), or scanning electron microscopy (SEM); electrochemical methods, such as measurement of impedance, voltage, or current; optical methods, such as confocal and nonconfocal microscopy, infrared microscopy, detection of fluorescence, luminescence, chemiluminescence, or absorbance, reflectance, or transmittance detection, and, in general, any surface analysis technique.

In another embodiment of the invention, detection of the hybridized fragments is accomplished by detection of labeling elements incorporated in the fragments to be analyzed. In particular, the labeling takes place during the *in vitro* transcription step by the incorporation of nucleotide analogs containing directly-detectable labeling, such as fluorophores, nucleotide analogs incorporating labeling that can be visualized indirectly in a subsequent reaction, such as biotin or haptenes, or any other type of direct or indirect nucleic acid labeling known to a person skilled in the art. In particular, the labeling can be performed using Cy3-UTP, Cy5-UTP, or fluorescein-UTP for direct labeling, or biotin-UTP for indirect labeling.

The expression functional promoter sequence refers to a nucleotide sequence that can be recognized by an RNA polymerase and from which transcription can be initiated. In general, each RNA polymerase recognizes a specific sequence, for which reason the functional promoter sequence included in the adapters is chosen according to the RNA polymerase being used. Examples of RNA polymerase include, but are not limited to, T7 RNA polymerase, T3 RNA polymerase, and SP6 RNA polymerase.

Also a kit comprising the reagents, enzymes and additives needed to accomplish the method of analyzing nucleic acids is described.

Further, a kit comprising the reagents, enzymes, additives and DNA microarrays with appropriate probes needed to accomplish the method of analyzing nucleic acids is disclosed herein.

The present invention is based on improving the methods of analyzing nucleic acids by the use of DNA microarrays to study variations in the genome of an organism that are in use at the present time. It was observed that when the preparation of DNA is associated with a step comprising *in vitro* transcription of PCR-amplified DNA fragments using an RNA polymerase, the signal to hybridize to the probes contained in the DNA microarray was stronger and more homogeneous than when DNA fragments obtained or labeled by other means were hybridized directly. Given that other supposedly random labeling methods result in a very substantial skewing of the efficacy of labeling and/or hybridization of the various labeled fragments, this result was unexpected, and indeed the reasons why the present invention reduces or eliminates this skewing are at present unknown.

To determine the improvement in the method of the present invention in comparison to other methods commonly used at the present time, the reference parameter used was the signal intensity scatter of the analyzed samples versus the signal intensity scatter of the hybridization controls.

For example, for each read channel of the scanner (corresponding to a given labeling) the relative percentage scatter of the signal intensities was calculated as the ratio between the standard deviation of the set of values and the mean of said values. In the examples of the present invention there are shown the data corresponding to labeling with Cy3 (green channel) and labeling with Cy5 (red channel). This calculation was done for both the probes and the controls included in the experiment, which also made it possible to calculate the ratio between the relative percentage scatter of the probe signals and the relative percentage scatter of the control signals. In this way a value is obtained that reflects the degree of scatter of the probes in comparison to the degree of scatter of the controls, given that the latter is indicative of the intrinsic variability of hybridization. Furthermore, the average ratio between the signal intensity of each point and the intensity and its own background noise was treated as another reference value. In all these calculations it may be expected that any approximation of normalization will affect all the values in a similar way, leaving the ratio more or less invariable.

To perform the comparative analysis of the signal intensities of the two samples, the intensity values obtained from hybridization to each probe in the microarray is usually represented in a logarithmic scatter plot representing the values from the first sample on the x-axis and the corresponding values for the second sample on the y-axis. The plot diagonal is represented by those points at which a given probe presents the same value for both samples. When comparing two identical samples, the points should ideally be located on the diagonal. It is observed experimentally, however, that a certain scatter of the points with respect to the diagonal occurs (i.e. a scatter perpendicular to the diagonal), or, therefore, a scatter of the ratio between the intensity values of two identical samples. This scatter is indicative of the degree of reproducibility of the data from one sample for each probe, and associated with it is a given standard deviation, calculated on the basis of the ratio between the signals from the two samples for the various probes on the surface.

Moreover, in the scatter plot described earlier, the hybridization signals for each of the probes are distributed along the diagonal (or parallel to the diagonal), said distribution being intrinsic to the signal intensity in a sample. This scatter reflects the variation in the detection efficiency of the various fragments in a sample, which is given by the variation in the efficiency of the protocol for the preparation of the various nucleic acid fragments for hybridization (including labeling, if applicable) combined with the variation in the efficiency of hybridization of the various nucleic acid fragments on the surface. This distribution along the entire signal intensity range has associated with it a relative standard deviation, defined as the ratio between the standard deviation of the intensities of the probes of a sample divided by the average of the intensities of all the probes for this sample. The relative standard deviation of the intensities can be calculated for the set of all the probes of the sample, or for a set of repeat probes acting as controls. The ratio between the standard deviation of the intensities of the sample divided by the ratio of the standard deviation of then intensities of the controls will therefore reflect the contribution of the variation in the efficiency of the protocol for the preparation of the various nucleic acid fragments for hybridization (including labeling, if applicable) and of the variation in the efficiency of hybridization of the various nucleic acid fragments on the surface to the total signal intensity scatter of a sample.

The present invention describes a protocol for the preparation of nucleic acid fragments which reducers the scatter of signal intensities from a sample obtained by their hybridization to DNA microarrays.

In one embodiment of the invention, hybridization to DNA microarrays and detection fulfills the requirement that, when all the analyzed fragments in the original nucleic acid were present in the same number of copies, the ratio between the relative scatter of the signal intensities of the probes of the sample and the relative scatter of the signal intensities of the controls is less than 4, preferably less than 3, more preferably less than 2, more preferably still less than 1.5.

One of the ways of controling the intensity of the hybridization signals along the diagonal is by varying the quantity of hybridized sample, such that the greater the quantity of hybridized sample, the greater the signal. In this way the maximum and minimum signals can be adjusted so that they are included within the detection range of the scanner. However, varying the quantity of sample applied does not affect the signal distribution profile: increasing the sample quantity in order to raise the intensity of low-intensity signals or signals that are below the detection threshold defined by the noise level of the analysis, will have the consequence that high-intensity signals will pass into the saturation region. Applying the method of analysis of the present invention results in a homogenization of the signal intensities of the probes of a sample, but also in an increase in the average signal intensity, and therefore improves the signal-to-noise ratio in the analyses.

In the method of the present invention, the sample hybridized to the DNA microarray is made up of RNA, which has certain advantages with respect to other methods. Firstly, the RNA-DNA interaction is stronger than the DNA-DNA interaction, which may be one reason for the observed increase in the average signal intensity. Secondly, the single-chained RNA does not have any competition from complementary molecules present in solution for hybridization to the probes on the microarray surface, resulting in a greater degree of hybridization to the probes contained in the DNA microarray surface.

Therefore, the present invention provides a new method of analyzing nucleic acids for the identification of variations in complex genomes with better sensitivity, signal-to-noise ratio, and reproducibility than protocols currently used.

### BRIEF DESCRIPTION OF THE FIGURES

Figure 1 presents a detailed diagram of an example of the stages involved in the method of the invention when using two restriction enzymes for digestion of the DNA sample.

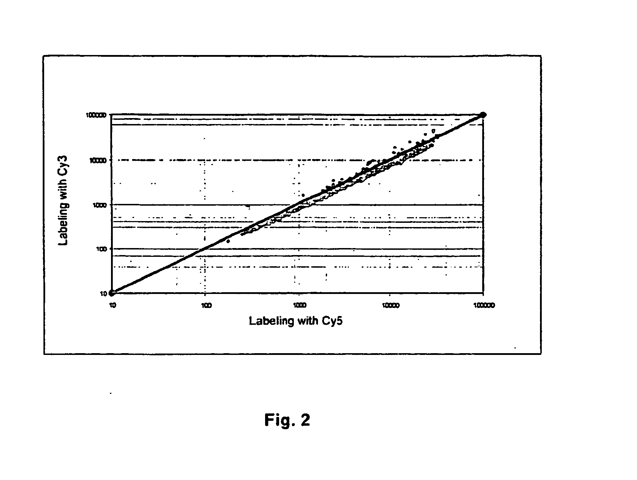

Figure 2 shows a logarithmic-scale graphical representation of the results obtained after analysis of yeast genomic DNA by the method as described in Example 1, i.e. with labeling of the samples during the PCR-amplification stage and without carrying out the *in* vitro transcription step. It is observed that the signal intensity values present a distribution along the plot diagonal.

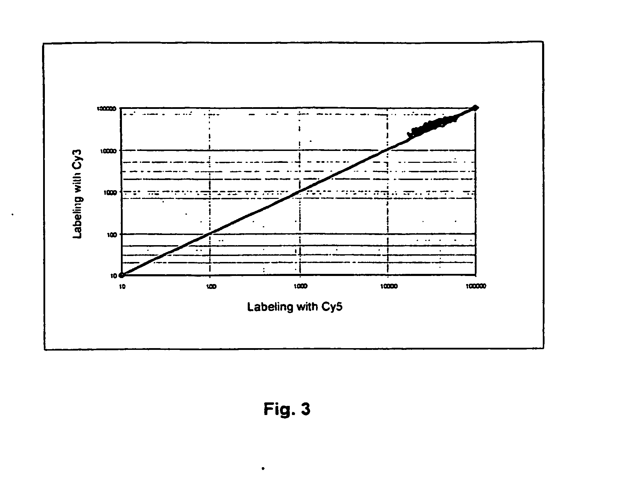

Figure 3 shows a logarithmic-scale graphical representation of the results obtained after analysis of yeast genomic DNA by the method of the invention, including an *in vitro* transcription step, as described in Example 2. It is observed that the signal intensity values exhibit a smaller distribution range when a labeling step is carried out in accordance with the method of the present invention.

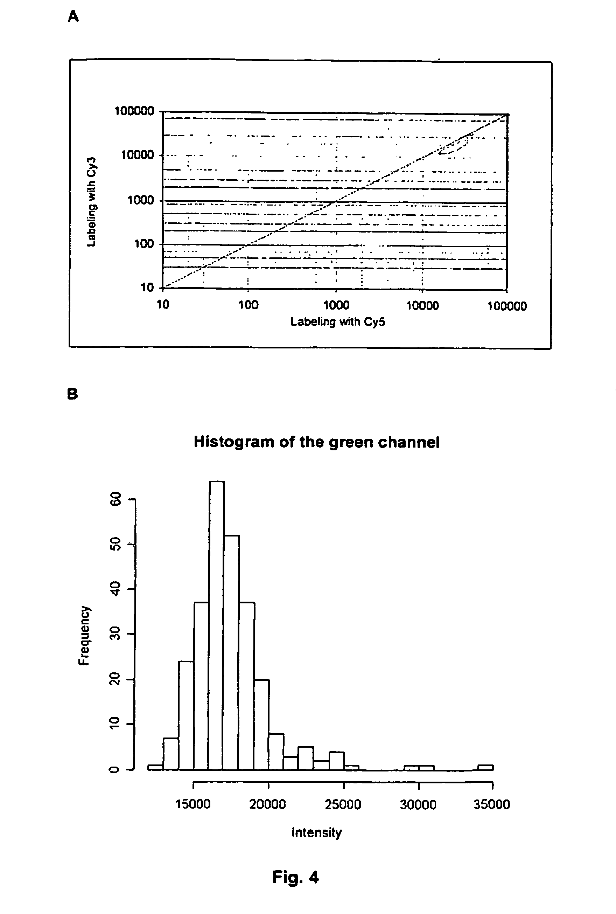

Figure 4A shows a logarithmic-scale graphical representation of the results obtained after analysis of rice genomic DNA by the method of the invention, as described in Example 3. Again it is observed that the signal intensity values exhibit a smaller distribution range when the method of the present invention is applied. Figure 4B shows the histogram corresponding to the frequency of the signal intensities obtained in Example 3 for the green channel, corresponding to labeling with Cy3. It is observed that the samples exhibit a normal distribution.
Figure 5A shows a logarithmic graphical representation of the data corresponding to DataSet14 from the study by Barrett *et al.* described above. The signal intensity values are observed to be distributed along the plot diagonal. Figure 5B shows the histogram corresponding to the frequency of the signal intensities for the same green channel data, corresponding to labeling with Cy3. It is observed that a greater signal intensity scatter is obtained, as well as a greater frequency at low signal intensities.

### EXAMPLES

Below are described some non-limiting examples of the method of the present invention.

### Example 1: Analysis of yeast genomic DNA with labeling by amplification with primers labeled with Cy3 and Cy5 fluorophores without an in vitro transcription step.

### DNA preparation

Genomic DNA was extracted from a species of yeast, *Saccharomyces cerevisiae.* Cells of the yeast culture were precipitated by centrifuging, resuspended in 600 µL of DNA extraction solution (100 mM Tris-HCl; 50 mM EDTA pH 8); 40 µL of 20% SDS was then added and the whole was mixed well and incubated for 10 min at 65°C; next, 200 µL of cold potassium acetate was added and incubation continued for 15 min on ice. The mixture was then centrifuged in a microcentrifuge at 4°C and 16000 rpm for 15 min, and 600 µL of isopropanol was added to 400 µL of supernatant. The DNA was precipitated by centrifuging at 16000 rpm for 15 min, after which the precipitate was washed with 200 µL of 70% ethanol and left to dry. The precipitate was dissolved in 100 µL of TE.

### DNA purification

2µL of RNase (10 mg/mL) was added to the sample and this was incubated for 15 min in a water bath at 37°C. 100 µL of cetyltrimethylammonium bromide (CTAB) solution was added (2% wt/vol CTAB; 200 mM Tris; 50 mM EDTA pH 7.5; 2 M NaCl), and after incubating for 15 min at 65°C, 200 µL of 24:1 CHCl₃ isoamyl alcohol was added. The mixture was centrifuged in the microcentrifuge for 5 min at 15000 rpm and 200 µL of the supernatant was precipitated with 180 µL of isopropanol. This was centrifuged in the microcentrifuge at 15000 rpm for 10 min, the precipitate was washed with 100 µL of 70% ethanol, and left to dry in air. Finally, the precipitate was dissolved in 50 µL of water.

### DNA digestion and adapter binding

Total genomic DNA (2 µg) was digested with Sacl (Fermentas, Lithuania) and Msel (New England Biolabs, USA) in an incubation time of 3 h at 37°C. To the DNA fragments generated by digestion, the Sacl adapter compatible with the cohesive end of the Sacl enzyme and the Msel adapter compatible with the Msel cohesive end were bound with T4 DNA ligase (Fermentas, Lithuania) in T4 ligase buffer (Fermentas, Lithuania) in an incubation time of 4 h at ambient temperature.

### DNA amplification

The Sacl/Msel fragments were amplified by PCR using two specific primers, based on the sequence of the adapters, each at a concentration of 200 nM, in a reaction with 1x Taq buffer, 1.5 nM of MgCl₂, 200 nM of dNTP, and 1. U of Taq polymerase (Fermentas, Lithuania), using the following cycle program: 2 min at 72°C; 2 min at 94°C; 34 cycles of 30 sec at 94°C, 30 sec at 56°C, 90 sec at 72°C, and 10 min at 72°C. In this case, one of the primers, the one specific for the Sacl adapter, was labeled. In this way the incorporation of the labeling was done as DNA amplification progressed in the PCR. The PCR was performed in duplicate, in parallel, such that in one case the primer contained one molecule of the fluorochrome Cy3 on the 5' end, while in the other case it contained the fluorochrome Cy5.

### Microarray hybridization

0.75 µg of DNA from the Cy3-labeled sample was combined with 0.75 µg of DNA from the Cy5-labeled sample and denatured at 98°C for 5 min before being hybridized. To this DNA mixture was added 100 µL of 2X hybridization solution (Agilent, USA) and the microarray hybridization was performed according to the recommendations of Agilent Technologies, USA. This hybridization consisted in overnight incubation at 60°C in a hybridization oven and subsequent washing with solutions 6 x SSC, 0.005% Triton (Agilent, USA) at ambient temperature, and 0.1 x SSC, 0.005% Triton (Agilent, USA) at 4°C to remove excess unhybridized transcripts with the microarray oligonucleotides. The microarray was then dried by centrifuging at 2000 rpm for 7 min and, finally, the intensity signals of each oligonucleotide in the microarray were detected with the Axon 4000B scanner.

The data obtained from reading the signal intensities for each of the fluorophores were represented graphically as shown in Figure 2. The signal intensities are observed to be distributed along the graph diagonal, in a similar manner to that which would be obtained in a differential expression analysis experiment, which indicates that there is variability in the labeling of the samples.

In addition, these data were processed for purposes of conducting a quantitative analysis. The relative percentage scatter of the signals was calculated, for both the probes and the controls included in the experiment, as a ratio of the standard deviation for each group of values and the mean of the said values. Also calculated was the ratio of the relative percentage scatter of the signals from the probes and the relative percentage scatter of the signals from the controls. This value reflects the degree of scatter of the probes in comparison to the scatter of the controls. The average ratio between the signal intensity at each point and the intensity of its own background noise was likewise calculated. The results are assembled in Table 2.

**Table 2**

| | Green channel | Red channel |
|---|---|---|
| Relative percentage scatter of the probe signals | 120% | 122% |
| Relative percentage scatter of the control signals | 26% | 26% |
| Ratio between probe scatter and control scatter | 4.61 | 4.69 |
| Average signal intensity with respect to background noise | 77 | 70 |

The results show that, owing to the variability in the labeling of the samples, the signals of the probes exhibit a scatter up to almost 5 times that of the controls included in the experiment.

### Example 2: Analysis of yeast genomic DNA with labeling by means of an in vitro transcription step.

### DNA preparation

Genomic DNA was extracted from a species of yeast, *Saccharomyces cerevisiae.* Cells of the yeast culture were precipitated by centrifuging, resuspended in 600 µL of DNA extraction solution (100 mM Tris-HCl; 50 mM EDTA pH 8); 40 µL of 20% SDS was then added and the whole was mixed well and incubated for 10 min at 65°C; next, 200 µL of cold potassium acetate was added and incubation continued for 15 min on ice. The mixture was then centrifuged in a microcentrifuge at 4°C and 16000 rpm for 15 min, and 600 µL of isopropanol was added to 400 µL of supernatant. The DNA was precipitated by centrifuging at 16000 rpm for 15 min, the precipitate was washed with 200 µL of 70% ethanol, and left to dry. The precipitate was dissolved in 100 µL of TE.

### DNA purification

2 µL of RNase (10 mg/mL) was added to the sample and this was incubated for 15 min in a water bath at 37°C. 100 µL of cetyltrimethylammonium bromide (CTAB) solution was added (2% wt/vol CTAB; 200 mM Tris; 50 mM EDTA pH 7.5; 2 M NaCl), and after incubating for 15 min at 65°C, 200 µL of 24:1 CHCl₃:isoamyl alcohol was added. The mixture was centrifuged in a microcentrifuge for 5 min at 15000 rpm and 200 µL of the supernatant was precipitated with 180 µL of isopropanol. This was centrifuged in the microcentrifuge at 15000 rpm for 10 min, the precipitate was washed with 100 µL of 70% ethanol, and left to dry in air. Finally, the precipitate was dissolved in 50 µL of water.

### DNA digestion and adapter binding

Total genomic DNA (2 µg) was digested with Sacl (Fermentas, Lithuania) and Msel (New England Biolabs, USA) in an incubation time of 3 h at 37°C. To the DNA fragments generated by digestion, the Sacl adapter compatible with the cohesive end of the Sacl enzyme and the Msel adapter compatible with the Msel cohesive end were bound with T4 DNA ligase (Fermentas, Lithuania) in T4 ligase buffer (Fermentas, Lithuania) in an incubation time of 4 h at ambient temperature.

### DNA amplification

The Sacl/Msel fragments were amplified by PCR using two specific primers, based on the sequence of the adapters, each at a concentration of 200 nM, in a reaction with 1x Taq buffer, 1.5 nM of MgCl₂, 200 nM of dNTP, and 1U of Taq polymerase (Fermentas, Lithuania), using the following cycle program: 2 min at 72°C; 2 min at 94°C; 34 cycles of 30 sec at 94°, 30 sec at 56°C, 90 sec at 72°C, and 10 min at 72°C.

### In vitro transcription

2.5 µg of PCR-amplified DNA was used to carry out the *in vitro* transcription to RNA from a promoter sequence contained in the Sacl adapter by the addition of 40 U of T7 RNA polymerase (Ambion, USA) and 7.5 mM of rNTPs, the sample being incubated overnight at 37°C. This reaction was performed in duplicate, in parallel, with Cy3-dUTP or Cy5-dUTP (Perkin-Elmer, USA) as labeled nucleotides. After transcription, the DNA was removed by treatment with 2 U of DNase I (Ambion, USA) at 37°C for 30 min. The labeled products were purified using MEGAclear™ columns (Ambion, USA).

### Microarray hybridization

0.75 µg of Cy3-labeled sample RNA was combined with 0.75 µg of Cy5-labeled sample RNA for hybridization to the microarray oligonucleotides. To this RNA mixture was added 100 µL of 2X hybridization solution (Agilent, USA) and loaded onto the chip as recommended by Agilent Technologies. Hybridization was accomplished overnight at 60°C in a hybridization oven. The microarray was then washed with solutions 6 x SSC, 0.005% Triton (Agilent, USA) at ambient temperature, and 0.1 x SSC, 0.005 Triton (Agilent, USA) at 4°C to remove excess unhybridized transcripts. Next, the chip was dried by centrifuging at 2000 rpm for 7 min and, finally, the intensity signals of each oligonucleotide in the microarray were detected with the Axon 4000B scanner.

The data obtained from reading the signal intensities for each of the fluorophores were represented graphically as shown in Figure 3. It is observed that the signal intensities are grouped in the upper part of the plot diagonal, which indicates that the labeling is more homogeneous than observed in Example 1, where the signals were distributed along the length of the diagonal.

In addition, the data were processed for purposes of conducting a quantitative analysis, as described in Example 1. The results are assembled in Table 3.

**Table 3**

| | Green channel | Red channel |
|---|---|---|
| Relative percentage scatter of the probe signals | 27% | 22% |
| Relative percentage scatter of the control signals | 23% | 36% |
| Ratio between probe scatter and control scatter | 1.17 | 0.61 |
| Average signal intensity with respect to background noise | 698 | 671 |

These results indicate that when a labeling step is performed by *in vitro* transcription according to the method of the present invention, the signals corresponding to the probes exhibit a scatter similar to that of the controls in the same experiment, in contrast to what happens when this step is not performed, as described in Experiment 1. This improvement in signal scatter makes it easier to detect those samples that could exhibit some alteration at genome level. Moreover, a better signal-to-noise ratio is also obtained.

### Example 3: Analysis of rice genomic DNA with labeling by means of an in vitro transcription step.

### DNA preparation

Genomic DNA was extracted from the rice species *Oryza sativa sp. japonica Nipponbare.* Plant leaf tissue frozen in liquid nitrogen was homogenized in a Mixer Mill (Retsch GmbH, Germany). The lysate resulting from the homogenization was resuspended in 600 µL of DNA extraction solution (100 mM Tris-HCl; 50 mM EDTA pH 8); 40 µL of 20% SDS was then added and the whole was mixed well and incubated for 10 min at 65 °C; next, 200 µL of cold potassium acetate was added and incubation continued for 15 min on ice. The mixture was then centrifuged in a microcentrifuge at 4°C and 16000 rpm for 15 min, and 600 µL of isopropanol was added to 400 µL of supernatant. The DNA was precipitated by centrifuging at 16000 rpm for 15 min, the precipitate was washed with 200 µL of 70% ethanol, and left to dry. The precipitate was dissolved in 100 µL of TE.

### Purification of DNA

2 µL of RNase (10 mg/mL) was added to the sample and this was incubated for 15 min in a water bath at 37°C. 100 µL of cetyltrimethylammonium bromide (CTAB) solution was added (2% wt/vol CTAB; 200 mM Tris; 50 mM EDTA pH 7.5; 2 M NaCl), and after incubating for 15 min at 65°C, 200 µL of 24:14 CHCl₃:isoamyl alcohol was added. The mixture was centrifuged for 5 min at 15000 rpm and 200 µL of the supernatant was precipitated with 180 µL of isopropanol. This was centrifuged in the microcentrifuge at 15000 rpm for 10 min, the precipitate was washed with 100 µL of 70% ethanol, and left to dry in air. Finally, the precipitate was dissolved in 50 µL of water.

### DNA digestion and adapter binding

Total genomic DNA (2 µg) was digested with Sacl (Fermentas, Lithuania) and Msel (New England Biolabs, USA) in an incubation time of 3 h at 37°C. To the DNA fragments generated by digestion, the Sacl adapter compatible with the cohesive end of the Sacl enzyme and the Msel adapter compatible with the Msel cohesive end were bound with T4 DNA ligase (Fermentas, Lithuania) in T4 ligase buffer (Fermentas, Lithuania) in an incubation time of 4 h at ambient temperature.

### DNA amplification

The Sacl/Msel fragments were amplified by PCR using two specific primers, based on the adapter sequence, each at a concentration of 200 nM, in a reaction with 1 x Taq buffer, 1.5 nM of MgCl₂, 200 nM of dNTP, and 1 U of Taq polymerase (Fermentas, Lithuania), using the following cycle program: 2 min at 72°C; 2 min at 94°C; 34 cycles of 30 sec at 94°, 30 sec at 56°C, 90 sec at 72°C, and 10 min at 72°C.

### In vitro transcription

2.5 µg of PCR-amplified DNA was used to carry out the *in vitro* transcription to RNA from a promoter sequence contained in the Sacl adapter by the addition of 40 U of T7 RNA polymerase (Ambion, USA) and 7.5 mM of rNTPs, the samples being incubated overnight at 37°C. This reaction was performed in duplicate, in parallel, with Cy3-dUTP or else Cy5-dUTP (Perkin-Elmer, USA) as labeled nucleotides. After transcription, the DNA was removed by treatment with 2 U of DNase I (Ambion, USA) at 37°C for 30 min. The labeled products were purified using MEGAclear™ columns (Ambion, USA).

### Microarray hybridization

0.75 µg of Cy3-labeled sample RNA was combined with 0.75 µg of Cy5-labeled sample RNA for hybridization to the microarray oligonucleotides. To this RNA mixture was added 100 µL of 2X hybridization solution (Agilent, USA) and loaded onto the chip as recommended by Agilent Technologies. Hybridization took place overnight at 60°C in a hybridization oven. The microarray was then washed with solutions 6 x SSC, 0.005% Triton (Agilent, USA) at ambient temperature, and 0.1 x SSC, 0.005% Triton (Agilent, USA) at 4°C to remove excess unhybridized transcripts. The chip was then dried by centrifuging at 2000 rpm for 7 min and, finally, the intensity signals of each oligonucleotide in the microarray were detected with the Axon 4000B scanner.

The data obtained from reading the signal intensities for each of the fluorophores were represented graphically, as shown in Figure 4A. As was observed in Example 2, the signals were again grouped in the upper part of the plot diagonal, indicating the small degree of scattering of same. In addition, Figure 4B shows the histogram of the signal intensity distribution in the green channel (corresponding to the Cy3 labeling), wherein a normal distribution can be observed, with most points lying in a central position within the intensity range (around 18000-19000 units of intensity), with the remaining points lying asymmetrically arranged above and below this central position.

In this case too, the data were processed for purposes of conducting a quantitative analysis, as described in Example 1. The results are assembled in Table 4.

**Table 4**

| | Green channel | Red channel |
|---|---|---|
| Relative percentage scatter of the probe signals | 15% | 13% |
| Relative percentage scatter of the control signals | 15% | 18% |
| Ratio between probe scatter and control scatter | 1.00 | 0.72 |
| Average signal intensity with respect to background noise | 550 | 352 |

In this case, the oligonucleotides ORY_C1_X80. ORY_C2_X70. ORY_C3 Z80, and ORY_C3_Z80 repeated 223 times on the microarray surface were used as internal controls.

These results confirm that when a genome like that of rice is analyzed - which is much more complex than the yeast genome analyzed in Example 2 - better results are obtained when applying the method of the present invention then when a labeling method is used that does not include a labeling step involving *in vitro* transcription.

## Claims

1. A method of analyzing nucleic acids for the study of genomic variations, comprising the following steps:
a) fragmentation of a sample of genomic DNA,
b) binding, at the ends of the DNA fragments obtained, of specific adapters compatible with the generated ends where at least one of the bound adapters contains a functional promoter sequence,
c) amplification of the fragments obtained using specific adapter-based primers,
d) *in vitro* transcription of the amplified DNA fragments with an RNA polymerase capable of initiating the transcription from a promoter sequence contained in the adapters using a mixture of nucleotides (rNTPs),
e) hybridization to DNA microarray oligonucleotides and detection of the hybridized fragments by detection of a labeling incorporated in step d) in the fragments to be analyzed by the incorporation of nucleotide analogs containing directly-detectable labeling, nucleotide analogs incorporating labeling that can be visualized indirectly by a subsequent reaction or any other type of direct or indirect nucleic acid labeling, and
f) quantitative comparison of the signals from various samples analyzed.

2. The method according to claim 1, wherein said DNA sample analyzed is a genomic DNA sample isolated from any organism wherein the study of the presence of genomic variations is desired.

3. The method according to claim 1 or 2, wherein said fragmentation of a sample of genomic DNA is accomplished by chemical methods, physical methods, or enzymatic methods.

4. The method according to claim 3, wherein said fragmentation of a genomic DNA sample is accomplished by digestion with at least one restriction enzyme, preferably by digestion with two restriction enzymes.

5. The method according to any of claims 1 to 4, wherein the DNA microarrays on which hybridization and detection are carried out comprise a collection of multiple immobilized oligonucleotides on a solid substrate, wherein each oligonucleotide is immobilized in a known position such that hybridization to each of the many oligonucleotides can be detected separately, wherein the substrate can be solid or porous, planar or nonplanar, unitary or distributed, and wherein the DNA microarrays can be manufactured with oligonucleotides deposited by any process or with oligonucleotides synthesized *in situ* by photolithography or by any other process.

6. The method according to any of claims 1 to 5, wherein said nucleotide analogs contain fluorophores as directly-detectable labeling, or biotin or haptenes as labeling that can be visualized indirectly by a subsequent reaction.

7. The method according to claim 6, wherein said nucleotide analog containing the labeling is Cy3-UTP, Cy5-UTP, or fluorescein-UTP for direct labeling or biotin-UTP for indirect labeling.

8. The method according to any of claims 1 to 7, wherein said detection of the hybridized fragments is accomplished on the basis of the direct quantification of the quantity of hybridized sample on the DNA probes contained in the DNA microarray, wherein said direct quantification can be accomplished by means of techniques that include, but are not limited to, atomic force microscopy (AFM), scanning tunneling microscopy (STM), or scanning electron microscopy (SEM); electrochemical methods, such as measurement of impedance, voltage, or current; or optical methods, such as confocal and nonconfocal microscopy, infrared microscopy, detection of fluorescence, luminescence, chemiluminescence, absorbance, reflectance, or transmittance.

9. The method according to any of claims 1 to 8, wherein said RNA polymerase used in the *in vitro* transcription step is selected from the group including, but not limited to, T7 RNA polymerase, T3 RNA polymerase, and SP6 RNA polymerase.

10. The method according to any of claims 1 to 9, wherein the requirement is met that the ratio between the relative scatter of the signal intensities of the sample probes and the relative scatter of the signal intensities of the controls be less than 4, preferably less than 3, more preferably less than 2, more preferably still less than 1.5.

## Patentansprüche

1. Verfahren zur Analyse von Nucleinsäuren für die Untersuchung von genomischen Variationen, umfassend die folgenden Schritte:
a) Fragmentierung einer Probe genomischer DNA,
b) Binden von spezifischen Adaptern an die Enden der erhaltenen DNA-Fragmente, wobei die spezifischen Adapter mit den erzeugten Enden kompatibel sind, wobei mindestens einer der gebundenen Adapter eine funktionale Promotorsequenz enthält,
c) Amplifikation der erhaltenen Fragmente unter Verwendung spezifischer Adapterbasierter Primer,
d) in vitro-Transkription der amplifizierten DNA-Fragmente mit einer RNA-Polymerase, die fähig ist, die Transkription von einer Promotorsequenz aus, die in den Adaptern enthalten ist, unter Verwendung einer Mischung von Nucleotiden (rNTPs) zu initiieren,
e) Hybridisierung von Oligonucleotiden an einen DNA-Microarray und Nachweis der hybridisierten Fragmente durch Nachweis einer Markierung, die in Schritt d) in die Fragmente, die analysiert werden sollen, eingebaut wurde durch den Einbau von Nucleotidanaloga, die direkt-nachweisbare Markierungen enthalten, von NucleotidAnaloga mit eingebauten Markierungen, die indirekt durch eine nachfolgende Reaktion visualisiert werden können, oder durch jede andere Art von direkter oder indirekter Nucleinsäuremarkierung, und
f) quantitativer Vergleich der Signale von verschiedenen analysierten Proben.

2. Verfahren gemäß Anspruch 1, wobei die analysierte DNA-Probe eine genomische DNA-Probe ist, die von einem beliebigen Organismus isoliert ist, bei dem die Untersuchung der Gegenwart von genomischen Variationen gewünscht ist.

3. Verfahren gemäß Anspruch 1 oder 2, wobei die Fragmentierung der Probe genomischer DNA durch chemische Verfahren, physikalische Verfahren oder enzymatische Verfahren erreicht wird.

4. Verfahren gemäß Anspruch 3, wobei die Fragmentierung einer genomischen DNA-Probe durch Verdau mit mindestens einem Restriktionsenzym erreicht wird, vorzugsweise durch Verdau mit zwei Restriktionsenzymen.

5. Verfahren gemäß einem der Ansprüche 1 bis 4, wobei die DNA-Microarrays, auf denen Hybridisierung und Nachweis ausgeführt werden, eine Sammlung von vielfachen immobilisierten Oligonucleotiden auf einem festen Substrat umfassen, wobei jedes Oligonucleotid auf einer bekannten Position immobilisiert wird, so dass die Hybridisierung zu jedem der vielen Oligonucleotide getrennt nachgewiesen werden kann, wobei das Substrat fest oder porös, eben oder uneben, einheitlich oder verteilt sein kann, und wobei die DNA-Microarrays mit Oligonucleotiden hergestellt werden können, die durch einen beliebigen Prozess angelagert werden, oder mit Oligonucleotiden, die in situ durch Photolithographie oder durch jeden anderen Prozess synthetisiert werden.

6. Verfahren gemäß einem der Ansprüche 1 bis 5, wobei die Nucleotidanaloga Fluorophore als direkt-nachweisbare Markierung enthalten oder Biotin oder Haptene als Markierung enthalten, die indirekt durch eine nachfolgende Reaktion visualisiert werden kann.

7. Verfahren gemäß Anspruch 6, wobei das Nucleotidanalogon, das die Markierung enthält, Cy3-UTP, Cy5-UTP oder Fluorescein-UTP für direkte Markierung oder Biotin-UTP für indirekte Markierung ist.

8. Verfahren gemäß einem der Ansprüche 1 bis 7, wobei der Nachweis der hybridisierten Fragmente auf Basis der direkten Quantifizierung der Menge der hybridisierten Probe auf den DNA-Sonden, die in dem DNA-Microarray enthalten sind, durchgeführt wird, wobei die direkte Quantifizierung durchgeführt werden kann mittels Techniken, die einschließen, aber nicht beschränkt sind auf, Atomkraftmikroskopie (AFM), Rastertunnelmikroskopie (STM) oder Rasterelektronenmikroskopie (SEM); elektrochemischen Verfahren, wie Messung der Impedanz, der Spannung oder des Stroms; oder optischen Verfahren, wie konfokaler und nicht-konfokaler Mikroskopie, Infrarotmikroskopie, Nachweise von Fluoreszenz, Lumineszenz, Chemilumineszenz, Absorptionsgrad, Reflexion oder Transmission.

9. Verfahren gemäß einem der Ansprüche 1 bis 8, wobei die RNA-Polymerase, die in dem *in vitro*-Transkriptionsschritt verwendet wird, ausgewählt ist aus der Gruppe einschließend aber nicht eingeschränkt auf T7-RNA-Polymerase, T3-RNA-Polymerase und SP6-RNA-Polymerase.

10. Verfahren gemäß einem der Ansprüche 1 bis 9, wobei das Erfordernis erfüllt ist, dass das Verhältnis zwischen der relativen Streuung der Signalintensitäten der Probensonden und der relativen Streuung der Signalintensitäten der Kontrollen weniger als 4, vorzugsweise weniger als 3, bevorzugter weniger als 2, noch bevorzugter weniger als 1,5 ist.

## Revendications

1. Procédé pour analyser des acides nucléiques afin d'étudier des variations génomiques, comprenant les étapes suivantes :
a) la fragmentation d'un échantillon d'ADN génomique,
b) la liaison, aux extrémités des fragments d'ADN obtenus, d'adaptateurs spécifiques compatibles avec les extrémités générées, où au moins un des adaptateurs liés contient une séquence promotrice fonctionnelle,
c) une amplification des fragments obtenus en utilisant des amorces spécifiques basées sur les adaptateurs,
d) une transcription *in vitro* des fragments d'ADN amplifiés avec une ARN polymérase capable d'initier la transcription à partir d'une séquence promotrice contenue dans les adaptateurs en utilisant un mélange de nucléotides (rNTP),
e) une hybridation à des oligonucléotides d'un microréseau d'ADN et la détection des fragments hybridés en détectant un marqueur qui est incorporé à l'étape d) dans les fragments devant être analysés par l'incorporation d'analogues de nucléotides contenant un marqueur directement détectable, d'analogues de nucléotides incorporant un marqueur qui peut être visualisé indirectement par une réaction ultérieure, ou un quelconque autre type de marquage d'acide nucléique direct ou indirect, et
f) une comparaison quantitative des signaux de divers échantillons analysés.

2. Procédé selon la revendication 1, dans lequel ledit échantillon d'ADN analysé est un échantillon d'ADN génomique isolé à partir d'un organisme quelconque dans lequel l'étude de la présence de variations génomiques est souhaitée.

3. Procédé selon la revendication 1 ou 2, dans lequel ladite fragmentation d'un échantillon d'ADN génomique est réalisée par des procédés chimiques, des procédés physiques, ou des procédés enzymatiques.

4. Procédé selon la revendication 3, dans lequel ladite fragmentation d'un échantillon d'ADN génomique est réalisée par une digestion avec au moins une enzyme de restriction, de préférence par une digestion avec deux enzymes de restriction.

5. Procédé selon l'une quelconque des revendications 1 à 4, dans lequel les microréseaux d'ADN sur lesquels l'hybridation et la détection sont réalisées comprennent une collection d'oligonucléotides multiples immobilisés sur un substrat solide, où chaque oligonucléotide est immobilisé au niveau d'une position connue de sorte qu'une hybridation à chacun des nombreux oligonucléotides puisse être détectée séparément, où le substrat peut être solide ou poreux, planaire ou non planaire, unitaire ou distribué, et où les microréseaux d'ADN peuvent être fabriqués avec des oligonucléotides déposés par un quelconque procédé ou avec des oligonucléotides synthétisés *in situ* par photolithographie ou par un quelconque autre procédé.

6. Procédé selon l'une quelconque des revendications 1 à 5, dans lequel les analogues de nucléotides contiennent des fluorophores en tant que marqueur directement détectable, ou la biotine ou des haptènes en tant que marqueur qui peut être visualisé indirectement par une réaction ultérieure.

7. Procédé selon la revendication 6, dans lequel ledit analogue de nucléotide contenant le marqueur est Cy3-UTP, Cy5-UTP, ou la fluorescéine-UTP pour un marquage direct ou la biotine-UTP pour un marquage indirect.

8. Procédé selon l'une quelconque des revendications 1 à 7, dans lequel ladite détection des fragments hybridés est réalisée en se basant sur la quantification directe de la quantité d'échantillon hybridé sur les sondes d'ADN contenues dans le microréseau d'ADN, où ladite quantification directe peut être réalisée au moyen de techniques qui comprennent, sans s'y limiter, la microscopie à force atomique (AFM), la microscopie à effet tunnel (STM), ou la microscopie électronique à balayage (SEM) ; des procédés électrochimiques, comme une mesure de l'impédance, de la tension ou du courant ; ou des procédés optiques comme la microscopie confocale ou non confocale, la microscopie infrarouge, la détection d'une fluorescence, d'une luminescence, d'une chimiluminescence, de l'absorbance, de la réflectance, ou de la transmittance.

9. Procédé selon l'une quelconque des revendications 1 à 8, dans lequel ladite ARN polymérase utilisée dans l'étape de transcription *in vitro* est sélectionnée dans le groupe comprenant, sans s'y limiter, l'ARN polymérase T7, l'ARN polymérase T3, et l'ARN polymérase SP6.

10. Procédé selon l'une quelconque des revendications 1 à 9, dans lequel l'exigence est satisfaite lorsque le rapport entre la dispersion relative des intensités de signal des sondes d'échantillon et la dispersion relative des intensités de signal des contrôles est inférieur à 4, de préférence inférieur à 3, de manière davantage préférée inférieur à 2, de manière encore davantage préférée inférieur à 1,5.
